# EUROPÄISCHE PATENTANMELDUNG

(11) **EP 3 770 792 A1**
(43) Veröffentlichungstag der Anmeldung: **27.01.2021**
(21) Anmeldenummer: 19188469.1
(22) Anmeldetag: 25.07.2019
(51) Int. Cl.: G06F 30/23, G06F 30/27, G06F 111/10, G06F 119/14, G01N 3/00

(54) **VERFAHREN UND VORRICHTUNG ZUR MODELLERSTELLUNG UND FESTIGKEITSBEWERTUNG VON SCHWEISSNÄHTEN ZWISCHEN MECHANISCHEN BAUTEILEN**

(71) Anmelder: 7tech GmbH, 3730 Eggenburg (AT)
(72) Erfinder: SCHLEGEL, Christoph, 3730 Eggenburg (AT)
(74) Vertreter: Horn Kleimann Waitzhofer Patentanwälte PartG mbB

(57) **Zusammenfassung**

Das Verfahren umfasst die folgenden Schritte:
a) Bereitstellen eines Finite-Elemente-Modells für die Baugruppe, bei welchem ein erstes Finite-Elemente-Netz für ein erstes Bauteil, ein getrenntes zweites Finite-Elemente-Netz für ein zweites Bauteil und ein drittes Finite-Elemente-Netz für eine das erste und das zweite Bauteil verbindende Schweißnaht aufweisend eine Anzahl von Kerben erzeugt werden, wobei das dritte Finite-Elemente-Netz eine Anzahl von weniger als 20 finiten Elementen im Querschnitt aufweist, die Kerben der Schweißnaht dabei scharfkantig modelliert sind und die Aufteilung der finiten Elemente einem definierten Netzmuster folgt,
b) Berechnen des Finite-Elemente-Modells, wobei von dem definierten Netzmuster des dritten Finite-Elemente-Netzes für die Schweißnaht Ergebnisgrößen der finiten Elemente und Knoten bereitgestellt werden, und
c) Anwenden eines auf das definierte Netzmuster des dritten Finite-Elemente-Netzes abgestimmten Kerbspannungs-Prognosealgorithmus zur Prognose auftretender Kerbspannungen in den Kerben unter Verwendung der bereitgestellten Ergebnisgrößen als Eingabeparameter.

## Beschreibung

Die vorliegende Erfindung betrifft ein computerimplementiertes Verfahren sowie eine computerimplementierte Vorrichtung zur Modellerstellung und Festigkeitsbewertung von Schweißnähten zwischen mechanischen Bauteilen einer Baugruppe unter Zuhilfenahme einer Finite-Elemente-Methode.

Das technische Gebiet der Erfindung betrifft die Modellerstellung und Festigkeitsbewertung von Schweißnähten zwischen mechanischen Bauteilen einer Baugruppe unter Zuhilfenahme einer Finite-Elemente-Methode.

CAE-Analysen (CAE, Computer-Aided-Engineering), die von einem Computer durchgeführt werden, sind weit verbreitet, um die Gebrauchstauglichkeit von technischen Strukturen zu simulieren und zu beurteilen. Eine gängige Methode von CAE-Analysen sind Finite-Elemente-Analysen. Dabei werden mechanische Bauteile mit finiten Elementen vernetzt und damit zum Beispiel Verformungen oder mechanische Spannungen unter gegebenen Lasten berechnet. Damit kann weiters zum Beispiel die Festigkeit einer Struktur bewertet werden.

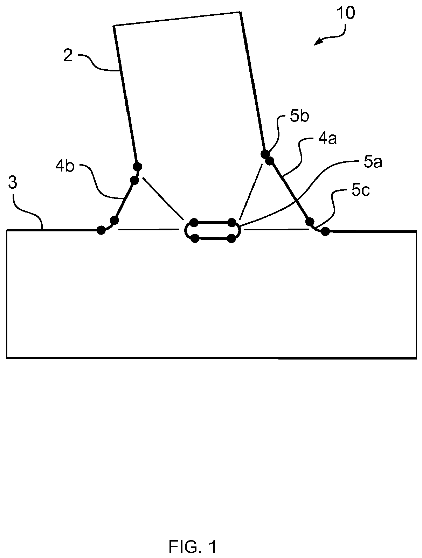

Werden Finite-Elemente-Modelle (FE-Modelle) berechnet, die Schweißnähte enthalten, liefern die berechneten Spannungen in den Schweißnähten nicht direkt eine Aussage über deren Tragfähigkeit. Einerseits bilden die FE-Modelle meist die Kerben (siehe Fig. 1) der jeweiligen Schweißnaht nicht genau genug ab, andererseits ist das Materialgefüge in der Schweißnaht nach dem Aufschmelzen und Abkühlen verändert und kann nicht wie das Grundmaterial bewertet werden.

Hierzu zeigt die Fig. 1 einen Querschnitt einer geschweißten Baugruppe 10. Die Bauteile 2 und 3 der Baugruppe 10 sind durch die Schweißnähte 4a und 4b verbunden. Für die Festigkeitsbewertung der Schweißnähte 4a, 4b mittels Kerbspannungskonzepten sind die Kerben 5a, 5b, 5c mit einem in entsprechenden Regelwerken definiertem Radius verrundet.

Es gibt eine Reihe von Regelwerken, die ergänzenden Methoden beschreiben, um auf Basis der FE-Ergebnisse Schweißnahtbewertungen durchzuführen. Die Methoden gliedern sich insbesondere in die folgenden drei Gruppen A, B und C:

### Gruppe A:

Bei Nennspannungsmethoden werden Kräfte und Momente an der Schweißnaht ausgewertet und damit Nennspannungen für einen Schweißnahtquerschnitt berechnet. Diese Nennspannungen müssen anschließend mit zulässigen Werten entsprechend einer Kerbfall-Klasse (FAT-Klasse) verglichen werden, welche der Anwender manuell wählen muss. Das erfordert gewisses Know-How und lässt fallweise Interpretationsspielraum, der zu Diskussionen und schwankenden Ergebnissen führt. Sonderfälle von Schweißnahtkonstellationen sind oft nicht in Standard-Kerbfall-Kathalogen enthalten.

### Gruppe B:

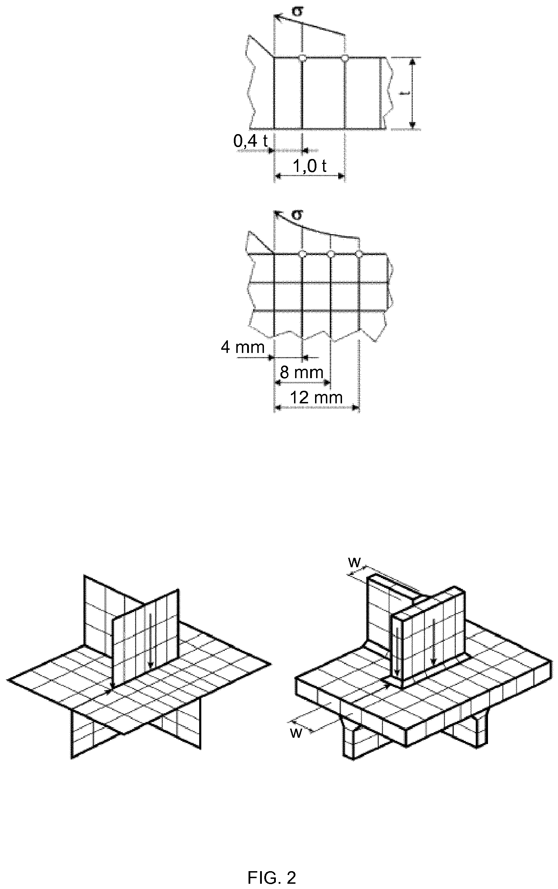

Fig. 2 zeigt Strukturspannungskonzepte. Dafür werden die Schweißnähte ausmodelliert und durchgängig (ohne Kontakt) vernetzt. Für die Bewertung werden Spannungen von Stützstellen im Grundmaterial (also neben der Schweißnaht) ausgewertet und in die Schweißnaht-Kerbe extrapoliert. Diese Spannungen werden dann wieder je nach Art der Schweißnaht mit Kerbfall- oder FAT-Klassen verglichen. Das bedeutet erheblichen Aufwand in der Modellerstellung und Auswertung. Und es erfordert wieder gewisses Know-How vom Anwender und lässt teilweise Interpretationsspielraum bei der Wahl der richtigen Kerbfall-Klasse. Die Abbildungen der Fig. 2 sind der IIW-Richtlinie gemäß Referenz [1] entnommen.

### Gruppe C:

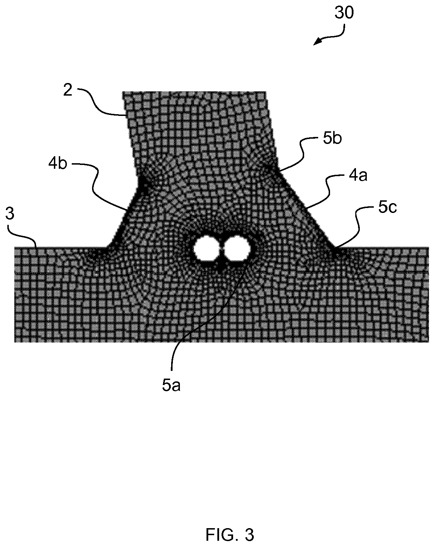

Am genauesten und allgemein gültigsten sind Kerbspannungsmethoden, wie in Fig. 3 dargestellt. In Fig. 3 zeigt Bezugszeichen 30 ein fein vernetztes FE-Modell einer Baugruppe mit zwei geschweißten Bauteilen 2, 3, wobei die jeweilige Schweißnaht 4a, 4b verrundete Kerben 5a, 5b, 5c aufweist. Aus Gründen der Übersichtlichkeit sind die Kerben nur für die rechte Schweißnaht 4a mit dem Bezugszeichen 5a, 5b, 5c versehen. Dabei müssen die Kerben 5a, 5b, 5c mit einem normierten Radius modelliert und sehr fein vernetzt werden. Auch das bedeutet erheblichen Modellierung- und Berechnungsaufwand. Die Bewertung läuft hier etwas einfacher, da die Kerbspannungen mit fixen normierten zulässigen Werten (z.B. FAT 225) verglichen werden. Diese Methode ist zwar für jede beliebige Schweißnahtkonstellation geeignet, lässt wenig Interpretationsspielraum und ist daher einfacher in der Anwendung. Aber da eine sehr feine Vernetzung und damit großer Rechenaufwand notwendig ist, wird die Kerbspannungsmethode typischerweise nur für kleine, sehr lokale Geometriedetails in sogenannten Submodellen verwendet. Für große Modelle mit vielen Schweißnähten kann die Kerbspannungsmethode wirtschaftlich nicht flächendeckend angewendet werden.

Kerbspannungs-Bewertungsmethoden sind zwar am universellsten einsetzbar und am genauesten, erfordern aber auch den meisten Rechenaufwand. Außerdem erfordern Kerbspannungsmethoden weniger Erfahrung und Know-How vom Anwender, da hier keine Schweißnaht-Kerbfälle oder Fatigue-Klassen aus Kathalogen gewählt werden müssen.

Alle herkömmlichen Methoden erfordern eine entsprechend gezielte Modellierung und Vernetzung. Nenn- und Strukturspannungsmethoden kommen mit FE-Modellen mit weniger Knoten und damit Rechenaufwand aus, benötigen aber mehr Benutzer-Input und Erfahrung und sind in der Ergebnisgenauigkeit schlechter als Kerbspannungsmethoden. Letztere sind sehr genau und eindeutig in der Anwendung, aber auch sehr rechenaufwändig.

Informationen zu herkömmlichen Methoden zur Modellerstellung und Festigkeitsbewertung von Schweißnähten zwischen mechanischen Bauteilen einer Baugruppe finden sich in den Referenzen [1] bis [9].

Vor diesem Hintergrund besteht eine Aufgabe der vorliegenden Erfindung darin, die Modellerstellung und Festigkeitsbewertung von Schweißnähten zwischen mechanischen Bauteilen einer Baugruppe zu verbessern.

Gemäß einem ersten Aspekt wird ein computerimplementiertes Verfahren zur Modellerstellung und Festigkeitsbewertung von Schweißnähten zwischen mechanischen Bauteilen einer Baugruppe unter Zuhilfenahme einer Finite-Elemente-Methode vorgeschlagen. Das Verfahren umfasst die folgenden Schritte:
a) Bereitstellen eines Finite-Elemente-Modells für die Baugruppe, bei welchem ein erstes Finite-Elemente-Netz für ein erstes Bauteil, ein getrenntes zweites Finite-Elemente-Netz für ein zweites Bauteil und ein drittes Finite-Elemente-Netz für eine das erste und das zweite Bauteil verbindende Schweißnaht aufweisend eine Anzahl von Kerben erzeugt werden, wobei das dritte Finite-Elemente-Netz eine Anzahl von weniger als 20 finiten Elementen im Querschnitt aufweist, die Kerben der Schweißnaht dabei scharfkantig modelliert sind und die Aufteilung der finiten Elemente einem definierten Netzmuster folgt,
b) Berechnen des Finite-Elemente-Modells, wobei von dem definierten Netzmuster des dritten Finite-Elemente-Netzes für die Schweißnaht Ergebnisgrößen der finiten Elemente und Knoten bereitgestellt werden, und
c) Anwenden eines auf das definierte Netzmuster des dritten Finite-Elemente-Netzes abgestimmten Kerbspannungs-Prognosealgorithmus zur Prognose auftretender Kerbspannungen in den Kerben (in einem verrundeten Zustand) unter Verwendung der bereitgestellten Ergebnisgrößen als Eingabeparameter.

Hierdurch werden die Berechnungen und Festigkeitsbewertung von Schweißnähten in Finite-Elemente-Modellen unter Verwendung von Kerbspannungs-Prognosealgorithmen erleichtert und beschleunigt. Hierbei wird es ermöglicht, die Schweißnähte scharfkantig zu modellieren und relativ grob zu vernetzen und trotzdem eine gute Prognose für die Kerbspannungen zu erhalten, mit denen anschließend eine Festigkeitsbewertung durchgeführt werden kann. Dies beschleunigt den Berechnungs- und Bewertungsprozess erheblich und ermöglicht ferner, auch bei FE-Modellen mit sehr vielen Schweißnähten, einen Kerbspannungs-Prognosealgorithmus anzuwenden. Die Modellierung und Auswertung der Schweißnähte ist vorteilhafterweise gut automatisierbar und von Software-Programmen ausführbar.

Da die Kerbspanungsmethode mit dem vorliegenden computerimplementierten Verfahren auch bei komplexen FE-Modellen herangezogen werden kann, braucht der Anwender weniger Know-How als bei herkömmlichen Methoden, welche die Wahl einer Kerbfallklasse erfordern. Des Weiteren kann der Anwender die genaue Art und die geometrischen Abmessungen der Schweißnähte anhand der Volumenvernetzung intuitiv kontrollieren und damit Fehler vermeiden. Dadurch, dass das Kerbspannungskonzept zugrunde gelegt ist, ist man mit der vorliegenden Methode auch nicht auf eine eingeschränkte Gruppe von Schweißnahttypen aus einem Kerbfallkatalog eingeschränkt, sondern kann jede beliebige Schweißkonstellation analysieren.

Wenn man Schweißnaht-Bewertungsmethoden kategorisieren möchte, kann man die vorgeschlagene Methode zwischen die bestehenden Strukturspannungs- und Kerbspannungsmethoden einordnen. Die vorgeschlagene Methode vereint durch die kleinere Knotenanzahl den geringeren Berechnungsaufwand der Strukturspannungsmethoden mit der allgemeiner gültigen Anwendbarkeit und der genaueren geometrischen Abbildung und leichteren Auswertbarkeit der Kerbspannungsmethoden.

Das dritte Finite-Elemente-Netz weist erfindungsgemäß eine Anzahl von 1 bis 20 finiten Elementen im Querschnitt auf. Eine geringe Elementanzahl führt zwar zu geringerer Berechnungsgenauigkeit, allerdings auch vorteilhafterweise zu kürzeren Rechenzeiten. Eine größere Elementanzahl und speziell kleinere Elemente in der Nähe der Schweißnahtkerben führen bei größerem Berechnungsaufwand und -zeit zu genaueren Ergebnissen. Die Methode kann also je nach Bedarf mehr in Richtung kürzere Berechnungszeit oder höhere Genauigkeit getrimmt werden.

Das finite Element ist insbesondere ein Volumenelement. Die finiten Elemente der Schweißnähte sind bei 3D-Modellen insbesondere als 3D-Volumenelemente und bei 2D-Modellen als 2D-Elemente ausgebildet und ergänzen das FE-Modell der ungeschweißten Bauteile.

Beispiele für mechanische Bauteile umfassen dünnwandige Teile, wie zum Beispiel Bleche oder Profile, oder dickwandige bzw. voluminöse Teile, wie zum Beispiel Gußteile.

Gemäß einer Ausführungsform werden das erste Finite-Elemente-Netz und das dritte Finite-Elemente-Netz mittels einer ersten Anzahl von Koppelelementen gekoppelt und das zweite Finite-Elemente-Netz und das dritte Finite-Elemente-Netz werden mittels einer zweiten Anzahl von Koppelelementen gekoppelt.

Durch die Verwendung der Koppelelemente ist keine gemeinsame durchgängige Vernetzung zwischen dem dritten Finite-Elemente-Netz für die Schweißnaht und dem ersten Finite-Elemente-Netz für das erste Bauteil notwendig. Entsprechend ist auch keine gemeinsame durchgängige Vernetzung zwischen dem dritten Finite-Elemente-Netz für die Schweißnaht und dem zweiten Finite-Elemente-Netz für das zweite Bauteil notwendig. Folglich kann das dritte Finite-Elemente-Netz nachträglich einem bestehenden und unverschweißten Bauteilnetz umfassend das erste Finite-Elemente-Netz und das zweite Finite-Elemente-Netz hinzugefügt werden. Zwischen den Finite-Elemente-Netzen für die Schweißnaht und für die Bauteile sind vorteilhafterweise keine gemeinsamen Knoten notwendig. Hierdurch ist der Aufwand für die Vernetzung vorteilhafterweise geringer. Auch kann das dritte Finite-Elemente-Netz für die Schweißnaht nachträglich eingebunden werden.

Gemäß einer weiteren Ausführungsform ist der Schritt b) ausgebildet durch:
b1) Berechnen des Finite-Elemente-Modells, und
b2) Auswerten der Ergebnisgrößen der finiten Elemente und der Knoten des definierten Netzmusters für die Schweißnaht auf der Basis des berechneten Finite-Elemente-Modells.

Gemäß einer weiteren Ausführungsform werden in dem Schritt b2) Ergebnisgrößen ausschließlich innerhalb der finiten Elemente und den Knoten des dritten Finite-Elemente-Netzes für die Schweißnaht ausgewertet.

Gemäß einer weiteren Ausführungsform umfassen die Ergebnisgrößen, welche in dem Schritt b2) ausgewertet werden:
- Spannungsergebnisse,
- Reaktionskraftergebnisse,
- Geometrieparameter, und/oder
- Materialparameter.

Gemäß einer weiteren Ausführungsform bestehen die Ergebnisgrößen, welche in dem Schritt b2) ausgewertet werden, aus:
- Spannungsergebnisse,
- Reaktionskraftergebnisse,
- Geometrieparameter, und
- Materialparameter.

Gemäß einer weiteren Ausführungsform wird der Kerbspannungs-Prognosealgorithmus vor der Anwendung des Schrittes c) mit einer Mehrzahl von Schweißnahtkonstellationen unter Verwendung des definierten Netzmusters trainiert.

Jede Schweißnahtkonstellation hat dabei vorzugsweise unterschiedliche geometrische Abmessungen (Parameter) der Bauteile und der Schweißnaht sowie unterschiedlichen Belastungen (Parameter) und stellt einen Designpoint im Parameterraum dar. Von jedem Designpoint wird vorzugsweise erstens die vorliegende Modellierungsmethode und zweitens eine Variante mit normgemäßem Kerb-Verrundungsradius und sehr feiner Vernetzung wie in Fig. 3 berechnet. Das zweite Modell liefert jeweils die Referenzergebnisse (Sollwerte) der Schweißnaht-Kerbspannungen und das erste Modell die Inputdaten für den Kerbspannungs-Prognosealgorithmus. Damit wird der Kerbspannungs-Prognosealgorithmus auf das vorliegende Vernetzungsmuster, den gegebenen Kerbradius und die vorliegende Modellierungsmethode des ersten Modells gefittet (trainiert). Der so trainierte Algorithmus kann anschließend bei produktiven Berechnungsmodellen angewendet werden, um Schweißnaht-Kerbspannungen zu prognostizieren.

Der Kerbspannungs-Prognosealgorithmus erfordert zwar in der Erstellung (beim Fitten bzw. Trainieren) einen gewissen Aufwand, ist aber in der nachfolgenden Anwendung sehr effizient und benötigt nur sehr geringe Rechenzeiten.

Gemäß einer weiteren Ausführungsform wird in dem Schritt c) eine Mehrzahl von Parametern der Kerben mittels des Kerbspannungs-Prognosealgorithmus prognostiziert.

Gemäß einer weiteren Ausführungsform umfassen diese Parameter:
- Hauptnormalspannungen,
- Schubspannungen,
- Von-Mises-Vergleichsspannungen, und/oder
- Radial-, Tangential- und/oder Axial-Spannungskomponenten im Kerbradius der jeweiligen Kerbe.

Gemäß einer weiteren Ausführungsform werden mit den prognostizierten Kerbspannungen anschließend Festigkeitsbewertungen der Baugruppe durchgeführt.

Gemäß einem zweiten Aspekt wird ein Computerprogrammprodukt vorgeschlagen, welches auf einer programmgesteuerten Einrichtung die Durchführung des wie oben erläuterten Verfahrens veranlasst.

Ein Computerprogrammprodukt, wie z.B. ein Computerprogramm-Mittel, kann beispielsweise als Speichermedium, wie z.B. Speicherkarte, USB-Stick, CD-ROM, DVD, oder auch in Form einer herunterladbaren Datei von einem Server in einem Netzwerk bereitgestellt oder geliefert werden. Dies kann zum Beispiel in einem drahtlosen Kommunikations-Netzwerk durch die Übertragung einer entsprechenden Datei mit dem Computerprogrammprodukt oder dem Computerprogramm-Mittel erfolgen.

Gemäß einem dritten Aspekt wird eine computerimplementierte Vorrichtung zur Modellerstellung und Festigkeitsbewertung von Schweißnähten zwischen mechanischen Bauteilen einer Baugruppe unter Zuhilfenahme einer Finite-Elemente-Methode vorgeschlagen. Die Vorrichtung umfasst:
eine erste Einheit zum Bereitstellen eines Finite-Elemente-Modells für die Baugruppe, bei welchem ein erstes Finite-Elemente-Netz für ein erstes Bauteil, ein getrenntes zweites Finite-Elemente-Netz für ein zweites Bauteil und ein drittes Finite-Elemente-Netz für eine das erste und das zweite Bauteil verbindende Schweißnaht aufweisend eine Anzahl von Kerben erzeugt werden, wobei das dritte Finite-Elemente-Netz eine Anzahl von weniger als 20 finiten Elementen im Querschnitt aufweist, die Kerben der Schweißnaht dabei scharfkantig modelliert sind und die Aufteilung der finiten Elemente einem definierten Netzmuster folgt,
eine zweite Einheit zum Berechnen des Finite-Elemente-Modells, wobei von dem definierten Netzmuster des dritten Finite-Elemente-Netzes für die Schweißnaht Ergebnisgrößen der finiten Elemente und Knoten bereitgestellt werden und
eine dritte Einheit zum Anwenden eines auf das definierte Netzmuster des dritten Finite-Elemente-Netzes abgestimmten Kerbspannungs-Prognosealgorithmus zur Prognose auftretender Kerbspannungen in den Kerben unter Verwendung der bereitgestellten Ergebnisgrößen als Eingabeparameter.

Die jeweilige Einheit kann hardwaretechnisch und/oder auch softwaretechnisch implementiert sein. Bei einer hardwaretechnischen Implementierung kann die Einheit als Vorrichtung oder als Teil einer Vorrichtung, zum Beispiel als Computer oder als Mikroprozessor, ausgebildet sein. Bei einer softwaretechnischen Implementierung kann die Einheit als Computerprogrammprodukt, als eine Funktion, als eine Routine, als Teil eines Programmcodes oder als ausführbares Objekt ausgebildet sein.

Die für das vorgeschlagene Verfahren beschriebenen Ausführungsformen und Merkmale gelten für die vorgeschlagene Vorrichtung entsprechend.

Weitere mögliche Implementierungen der Erfindung umfassen auch nicht explizit genannte Kombinationen von zuvor oder im Folgenden bezüglich der Ausführungsbeispiele beschriebenen Merkmale oder Ausführungsformen. Dabei wird der Fachmann auch Einzelaspekte als Verbesserungen oder Ergänzungen zu der jeweiligen Grundform der Erfindung hinzufügen.

Weitere vorteilhafte Ausgestaltungen und Aspekte der Erfindung sind Gegenstand der Unteransprüche sowie der im Folgenden beschriebenen Ausführungsbeispiele der Erfindung. Im Weiteren wird die Erfindung anhand von bevorzugten Ausführungsformen unter Bezugnahme auf die beigelegten Figuren näher erläutert.
- Fig. 1: zeigt einen Querschnitt einer geschweißten Baugruppe;
- Fig. 2: zeigt als erstes Beispiel für den Stand der Technik die Strukturspannungsmethode;
- Fig. 3: zeigt als zweites Beispiel für den Stand der Technik das Kerbspannungskonzept;
- Fig. 4: zeigt den Querschnitt eines FE-Modells einer Baugruppe mit erfindungsgemäß modellierter Schweißnaht;
- Fig. 5: zeigt den Querschnitt eines FE-Modells einer Baugruppe mit einer Variante einer erfindungsgemäß modellierten Schweißnaht;
- Fig. 6: zeigt den Querschnitt eines FE-Modells einer Baugruppe mit einer weiteren Variante einer erfindungsgemäß modellierten Schweißnaht;
- Fig. 7: zeigt Querschnitte von FE-Modellen von Baugruppen mit verschiedenen Varianten von erfindungsgemäß modellierten Schweißnähten;
- Fig. 8: zeigt verschiedene Anwendungsfälle von erfindungsgemäß modellierten Schweißnähten;
- Fig. 9: zeigt ein schematisches Ablaufdiagramm eines Ausführungsbeispiels eines computerimplementierten Verfahrens zur Modellerstellung und Festigkeitsbewertung von Schweißnähten zwischen mechanischen Bauteilen einer Baugruppe unter Zuhilfenahme einer Finite-Elemente-Methode; und
- Fig. 10: zeigt ein schematisches Blockschaltbild eines Ausführungsbeispiels einer computerimplementierten Vorrichtung zur Modellerstellung und Festigkeitsbewertung von Schweißnähten zwischen mechanischen Bauteilen einer Baugruppe unter Zuhilfenahme einer Finite-Elemente-Methode.

In den Figuren sind gleiche oder funktionsgleiche Elemente mit denselben Bezugszeichen versehen worden, sofern nichts anderes angegeben ist.

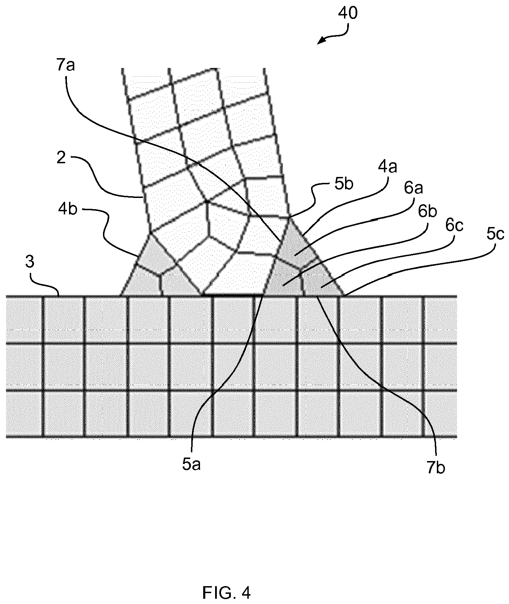

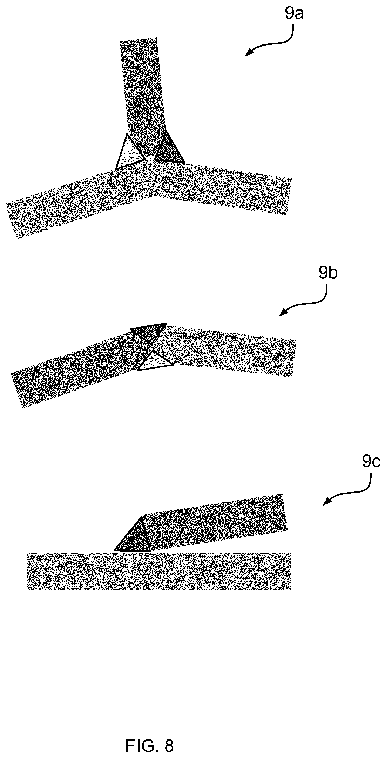

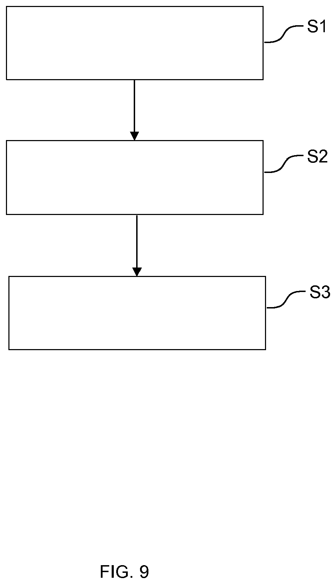

Ausführungsbeispiele zur Modellerstellung und Festigkeitsbewertung von Schweißnähten 4a, 4b zwischen mechanischen Bauteilen 2, 3 einer Baugruppe werden unter gemeinsamer Bezugnahme auf die Fig. 4 bis 9 erläutert. Dabei zeigen die Fig. 4 bis 7 Beispiele für FE-Modelle einer Baugruppe mit erfindungsgemäßer Schweißnaht. Ferner zeigt die Fig. 8 Anwendungsfälle von erfindungsgemäß modulierten Schweißnähten. Des Weiteren zeigt die Fig. 9 ein Ausführungsbeispiel eines computerimplementierten Verfahrens zur Modellerstellung und Festigkeitsbewertung von Schweißnähten 4a, 4b zwischen mechanischen Bauteilen 2, 3 einer Baugruppe unter Zuhilfenahme einer Finite-Elemente-Methode.

Beginnend mit Fig. 4 zeigt diese ein FE-Modell 40 einer geschweißten Baugruppe. Dabei sind ein erstes Bauteil 2 und ein zweites Bauteil 3 der Baugruppe mittels zweier Schweißnähte 4a, 4b geschweißt. Die Kerben der Schweißnaht 4a sind mit dem Bezugszeichen 5a, 5b, 5c versehen und die finiten Elemente, welche die Schweißnaht 4a abbilden, sind mit dem Bezugszeichen 6a, 6b, 6c versehen. Aus Gründen der Übersichtlichkeit sind nur die Kerben und die finiten Elemente der Schweißnaht 4a mit Bezugszeichen versehen, nicht aber die Kerben und die finiten Elemente der Schweißnaht 4b.

Die finiten Elemente 6a, 6b, 6c sind bei 3D-Modellen insbesondere als 3D-Volumenelemente und bei 2D-Modellen als 2D-Elemente ausgebildet und ergänzen das FE-Modell 40 des geschweißten Bauteils. Die Bauteile 2, 3 und die Schweißnähte 4a, 4b können dabei entweder mit gemeinsamen Knoten durchgängig gemeinsam oder unabhängig voneinander mit getrennten Knoten vernetzt werden. Eine getrennte, unabhängige Vernetzung hat den Vorteil, dass die Variation der Schweißnaht-Geometrie leichter möglich ist und dabei keine Änderung des Grundmodells der Bauteile 2, 3 notwendig ist.

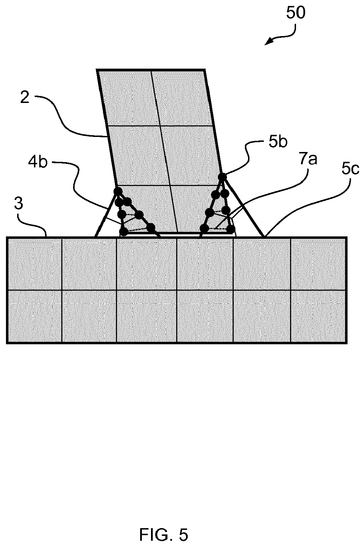

Bei unabhängiger Vernetzung werden die Schweißnahtelemente 6a, 6b, 6c mit den Bauteilen 2, 3 mithilfe von Koppelelementen 7a, 7b verbunden (siehe hierzu auch Fig. 5). Beispiele für Koppelelemente 7a, 7b umfassen unter anderem Kontaktelemente, Koppelbalken oder Koppelgleichungen.

Die unabhängige Vernetzung und Verbindung mit Koppelelementen 7a, 7b wird ermöglicht, da vorzugsweise Ergebnisgrößen nur von innerhalb der Schweißnahtelemente oder -knoten ausgewertet werden.

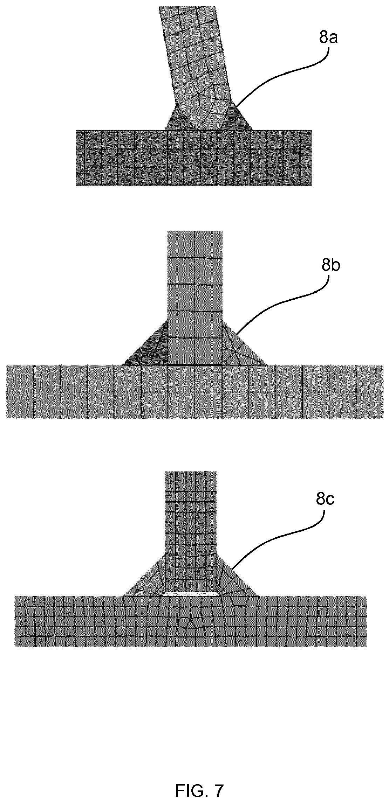

Die Schweißnähte 4a, 4b werden mit einem definierten Netzmuster vernetzt, wobei diese Netzmuster auf einen nachfolgend verwendeten Kerbspannungs-Prognosealgorithmus abgestimmt sind. Die Schweißnahtelemente 6a, 6b, 6c weisen vorzugsweise eine vordefinierte Anzahl, eine vordefinierte Aufteilung und eine vordefinierte Position innerhalb der Schweißnaht 4a, 4b auf. Die Kerben 5a, 5b, 5c der Schweißnaht 4a werden dabei nicht verrundet, sondern scharfkantig modelliert. Dies erlaubt eine relativ grobe Vernetzung und spart dadurch erheblich Berechnungsaufwand und Berechnungszeit ein. Die Geometrie, die Abmessungen und die Position der jeweiligen Schweißnaht 4a, 4b werden vorzugsweise realistisch modelliert, weshalb die Steifigkeit mit guter Genauigkeit abgebildet wird und das definierte Netzmuster wird an die gegebene Schweißnahtgeometrie proportional angepasst. Hierzu zeigt die Fig. 7 einige Beispiele für strukturierte Netzmuster 8a, 8b, 8c für Schweißnähte 4a, 4b. Anzumerken ist, dass der nachfolgend verwendete Kerbspannungs-Prognosealgorithmus auf das verwendete Netzmuster 8a, 8b, 8c abgestimmt ist. Das Erstellen einer Schweißnahtvernetzung mit definiertem Netzmuster 8a, 8b, 8c kann automatisiert mittels Softwareroutinen erfolgen (siehe hierzu Verfahrens schritt S1 der Fig. 9).

Das so aufbereitete FE-Modell der Baugruppe inklusive der Schweißnähte 4a, 4b wird anschließend mittels eines Berechnungsverfahrens gelöst und die Ergebnisse werden ausgewertet (siehe Verfahrensschritt S2 der Fig. 9).

Von den Schweißnähten 4a, 4b werden eine Reihe von Parametern ausgewertet und dem Kerbspannungs-Prognosealgorithmus als Inputdaten zur Verfügung gestellt. Die Parameter können Spannungen, Dehnungen und/oder Reaktionskräfte der Schweißnaht-Elemente 6a, 6b, 6c und Knoten sein. Zusätzlich können Material- und/oder Geometrieparameter, wie zum Beispiel die Abmessungen des Schweißnahtquerschnittes, relative Positionskoordinaten einzelner Knoten innerhalb des Schweißnahtquerschnittes oder Anschlußwinkel der Anschlußgeometrie in den einzelnen Schweißnaht-Querschnitten und -Kerben, verwendet werden.

Der Kerbspannungs-Prognosealgorithmus umfasst insbesondere Metamodelle oder Response-Surface-Methoden, wie zum Beispiel:
- Globale Polynome
- Moving least squares
- Kriging
- Radial basis functions
- Neuronale Netze

Diese Modelle werden beispielsweise erstellt (gefittet bzw. trainiert) mittels:
- Regression
- Interpolation
- Extrapolation

Die Kerbspannungs-Prognosealgorithmen werden jeweils auf eine gegebene Schweißnaht-Modellierungsmethode mit gegebenem Netzmuster gefittet (trainiert). Inputdaten des Kerbspannungs-Prognosealgorithmus sind eine relevante Untergruppe der oben genannten Parameter. Outputdaten sind Kerbspannungen und Kerbspannungskomponenten für jede Schweißnahtkerbe je SchweißnahtQuerschnitt.

Um den Kerbspannungs-Prognosealgorithmus fitten (trainieren) zu können, wird vorzugsweise eine ausreichende Anzahl von Schweißnahtkonstellationen berechnet. Jede Schweißnahtkonstellationen hat unterschiedliche geometrische Abmessungen (Parameter) der Bauteile und der Schweißnaht sowie unterschiedlichen Belastungen (Parameter) und stellt einen Designpoint im Parameterraum dar. Von jedem Designpoint wird vorzugsweise erstens die vorliegende Modellierungsmethode und zweitens eine Variante mit normgemäßem Kerb-Verrundungsradius und sehr feiner Vernetzung wie in Fig. 3 berechnet. Das zweite Modell liefert jeweils die Referenzergebnisse (Sollwerte) der Schweißnaht-Kerbspannungen und das erste Modell die Inputdaten für den Kerbspannungs-Prognosealgorithmus. Damit wird der Kerbspannungs-Prognosealgorithmus auf das vorliegende Vernetzungsmuster, den gegebenen Kerbradius und die vorliegende Modellierungsmethode des ersten Modells gefittet (trainiert). Der so trainierte Algorithmus kann anschließend bei produktiven Berechnungsmodellen angewendet werden, um Schweißnaht-Kerbspannungen zu prognostizieren. Auch wenn die Prognosegenauigkeit etwas schlechter sein kann als bei der klassischen verrundeten und fein vernetzten Kerbspannungsberechnungsmethode, ergibt die erfindungsgemäße Methode trotzdem einen enormen Vorteil, da mit wesentlich weniger Knoten erheblich kürzere Berechnungszeiten erzielt werden bzw. es überhaupt erst ermöglicht wird, dass Kerbspannungsbewertungsmethoden an komplexen Finiten-Elemente-Modellen mit vielen Schweißnähten wirtschaftlich angewendet werden können. Ohne das vorliegende Verfahren wäre bei komplexen Modellen die Elemente- und Knotenanzahl für Kerbspannungsberechnung zu groß, um wirtschaftlich berechnet werden zu können.

Mit den so prognostizierten Kerbspannungen und Kerbspannungskomponenten kann in weiterer Folge eine Betriebs- oder Dauerfestigkeitsbewertung der Schweißnaht durchgeführt werden.

Diese Kerbspannungs-Bewertungsmethode wird jeweils für einen Querschnitt einer Schweißnaht angewendet, das heißt in Schweißnaht-Längsrichtung können damit in definierten Abständen jeweils neue lokale Kerbspannungen prognostiziert werden.

Die Modellierungsmethode kann in gleicher Art und Weise für unterschiedliche Schweißnaht-Anwendungsfälle eingesetzt werden. Fig. 8 zeigt die Einsatzmöglichkeit für T-Stöße 9a, Stumpfstöße 9b und Überlapp-Stöße 9c. Für zweiseitig geschweißte Verbindungen wird vorzugsweise je Seite ein Schweißnahtmodell verwendet. Der Kerbspannungs-Prognosealgorithmus kann vorzugsweise so gefittet werden, dass er für alle diese Einsatzfälle unverändert eingesetzt werden kann. Für höhere Prognosegenauigkeit können aber für einzelne Einsatzfälle auch spezialisierte Kerbspannungs-Prognosealgorithmen gefittet werden.

Wie zum Beispiel in Fig. 7 - 8b und 8c gezeigt, werden bei einfachen Schweißnaht-Kehlnähten Bauteile ohne geometrischer Schweißnahtvorbereitung verschweißt. Um eine bessere und durchgängige Verbindung zu erhalten, werden die Bauteile, wie in Fig. 8a dargestellt, auch häufig angeschrägt und so mit einer geometrischen Schweißnahtvorbereitung versehen. Fig. 4 zeigt eine erfindungsgemäß modellierte Schweißnaht bei der die Schweißnahtvorbereitung am Bauteil voll ausmodelliert und vernetzt ist. Es können, wie in Fig. 5 dargestellt, die Bauteile aber auch ohne Schweißnahtvorbereitung modelliert werden. Das wird durch die unabhängige Schweißnahtvernetzung und der Verbindung der Schweißnähte mit den Bauteilen über Koppelelemente oder Koppelgleichungen 7a ermöglicht. Das erleichtert die Variation der Schweißnahtgeometrie, ohne das Finite-Elemente-Modell der Bauteile selbst ändern zu müssen.

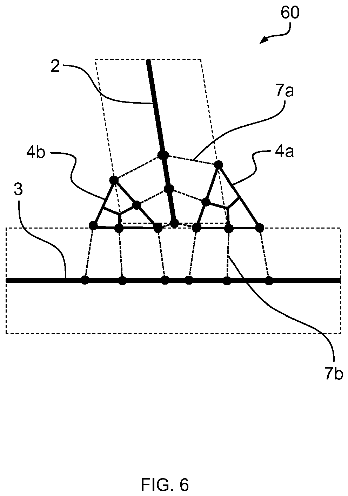

Die vorliegende Modellierungsmethode erlaubt auch, wie in Fig. 6 dargestellt, in gleicher Weise die Anwendung an Schalenmodellen 60. Die Bauteile 2, 3 werden dabei mit Finiten Schalenelementen in der Mittelebene der Bauteile 2, 3 und die Schweißnähte 4a, 4b mit dem definierten Netzmuster und der realen Schweißnahtgeometrie vernetzt. Die Verbindung erfolgt wieder mit Koppelelementen oder Koppelgleichungen 7a, 7b. Die vorliegende Modellierungs- und Kerbspannungs-Prognosemethode kann demnach in vielen verschiedenen Anwendungsfällen (Fig. 4, 5, 6, 8) verwendet werden.

Fig. 9 zeigt ein schematisches Ablaufdiagramm eines Ausführungsbeispiels eines computerimplementierten Verfahrens zur Modellerstellung und Festigkeitsbewertung von Schweißnähten 4a, 4b zwischen mechanischen Bauteilen 2, 3 einer Baugruppe unter Zuhilfenahme einer Finite-Elemente-Methode. Das Verfahren der Fig. 9 umfasst die Verfahrensschritte S1 bis S3 und wird unter Bezugnahme auf die Fig. 4 bis 8 erläutert:
In Schritt S1 wird ein Finite-Elemente-Modell 40 (siehe Fig. 4), 50 (siehe Fig. 5), 60 (siehe Fig. 6) für die Baugruppe bereitgestellt. Bei diesem Finite-Elemente-Modell 40, 50, 60 wird ein erstes Finite-Elemente-Netz für ein erstes Bauteil 2, ein getrenntes Finite-Elemente-Netz für ein zweites Bauteil 3 und ein drittes Finite-Elemente-Netz für eine das erste Bauteil 2 und das zweite Bauteil 3 verbindende Schweißnaht 4a, 4b aufweisend eine Anzahl von Kerben 5a, 5b, 5c erzeugt. Dabei weist das dritte Finite-Elemente-Netz eine Anzahl von weniger als 20 finiten Elementen 6a, 6b, 6c im Querschnitt auf. Die Kerben 5a, 5b, 5c der Schweißnaht 4a, 4b werden dabei scharfkantig modelliert. Die Aufteilung der finiten Elemente folgt dabei einem definierten Netzmuster 8a, 8b, 8c (siehe Fig. 8). Beispielsweise werden das erste Finite-Elemente-Netz und das dritte Finite-Elemente-Netz mittels einer Anzahl von Koppelelementen 7a, 7b, 7c gekoppelt und das zweite Finite-Elemente-Netz und das dritte Finite-Elemente-Netz werden mittels einer zweiten Anzahl von Koppelelementen 7a, 7b, 7c gekoppelt.

In Schritt S2 wird das Finite-Elemente-Modell 40, 50 ,60 berechnet, wobei von dem definierten Netzmuster 8a, 8b, 8c des dritten Finite-Elemente-Netzes für die Schweißnaht 4a, 4b Ergebnisgrößen der definierten Elemente und Knoten bereitgestellt werden.

Beispielsweise umfasst der Schritt S2 die folgenden Teilschritte:
- Berechnen des Finite-Elemente-Modells 40, 50, 60, und
- Auswerten der Ergebnisgrößen der finiten Elemente und der Knoten des definierten Netzmusters 8a, 8b, 8c für die Schweißnaht 4a, 4b auf der Basis des berechneten Finite-Elemente-Modells 40, 50, 60.

Dabei werden vorzugsweise Ergebnisgrößen ausschließlich innerhalb der finiten Elemente und den Knoten des dritten Finite-Elemente-Netzes für die Schweißnaht 4a, 4b ausgewertet. Die Ergebnisgrößen umfassen und bestehen vorzugsweise aus: Spannungsergebnisse, Reaktionskraftergebnisse, Geometrieparameter, und/oder Materialparameter.

In Schritt S3 wird ein Kerbspannungs-Prognosealgorithmus zur Prognose auftretender Kerbspannung in den Kerben 5a, 5b ,5c unter Verwendung der bereitgestellten Ergebnisgrößen als Eingabeparameter angewendet. Der Kerbspannungs-Prognosealgorithmus prognostiziert die auftretenden Kerbspannungen in den Kerben 5a, 5b ,5c in deren verrundeten Zustand. Der angewendete Kerbspannungs-Prognosealgorithmus ist auf das definierte Netzmuster 8a, 8b, 8c des dritten Finite-Elemente-Netzes abgestimmt.

Der Kerbspannungs-Prognosealgorithmus wird vorzugsweise vor dessen Anwendung mit einer Mehrzahl von Schweißnaht-Parametervarianten für die Schweißnaht 4a, 4b unter Verwendung des definierten Netzmusters 8a, 8b, 8c trainiert. Mittels des Kerbspannungs-Prognosealgorithmus wird eine Mehrzahl von Parametern prognostiziert. Diese Mehrzahl von Parametern umfasst: Hauptnormalspannungen, Schubspannungen, Von-Mises-Vergleichsspannungen, Radial-Tangential-Spannungskomponenten und/oder Axial-Spannungskomponenten im Kerbradius der jeweiligen Kerbe 5a, 5b, 5c.

Mit den prognostizierten Kerbspannungen können anschließend Festigkeitsbewertungen der Baugruppe durchgeführt werden.

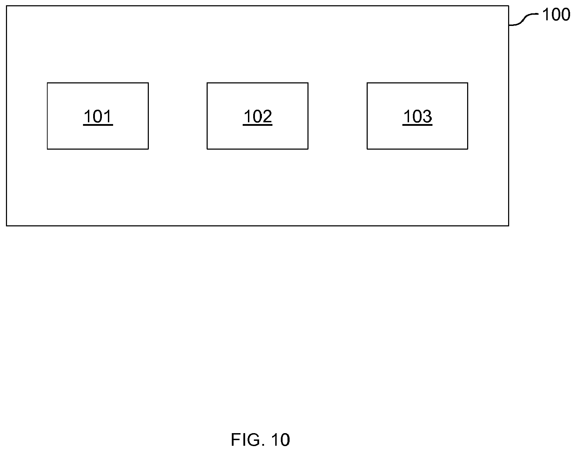

In Fig. 10 ist ein schematisches Blockschaltbild eines Ausführungsbeispiels einer computerimplementierten Vorrichtung 100 zur Modellerstellung und Festigkeitsbewertung von Schweißnähten 4a, 4b zwischen mechanischen Bauteilen 2, 3 einer Baugruppe unter Zuhilfenahme einer Finite-Elemente-Methode dargestellt.

Die Vorrichtung 100 umfasst eine erste Einheit 101, eine zweite Einheit 102 und eine dritte Einheit 103.

Die erste Einheit 101 ist zum Bereitstellen eines Finite-Elemente-Modells 40, 50, 60 für die Baugruppe eingerichtet, bei welchem ein erstes Finite-Elemente-Netz für ein erstes Bauteil 2, ein getrenntes zweites Finite-Elemente-Netz für ein zweites Bauteil 3 und ein drittes Finite-Elemente-Netz für eine das erste Bauteil 2 und das zweite Bauteil 3 verbindende Schweißnaht 4a, 4b aufweisend eine Anzahl von Kerben 5a, 5b, 5c erzeugt werden. Dabei weist das dritte Finite-Elemente-Netz eine Anzahl von weniger als 20 finiten Elementen 6a, 6b, 6c im Querschnitt auf, die Kerben 5a, 5b, 5c der Schweißnaht 4a, 4b sind dabei scharfkantig modelliert und die Aufteilung der finiten Elemente folgt einem definierten Netzmuster 8a, 8b, 8c.

Die zweite Einheit 102 ist zum Berechnen des Finite-Elemente-Modells 40, 50, 60 eingerichtet, wobei von dem definierten Netzmuster 8a, 8b, 8c des dritten Finite-Elemente-Netzes für die Schweißnaht 4a, 4b Ergebnisgrößen der finiten Elemente und Knoten bereitgestellt werden.

Die dritte Einheit 103 ist zum Anwenden eines auf das definierte Netzmuster 8a, 8b, 8c des dritten Finite-Elemente-Netzes abgestimmten Kerbspannungs-Prognosealgorithmus zur Prognose auftretender Kerbspannungen in den Kerben 5a, 5b, 5c unter Verwendung der bereitgestellten Ergebnisgrößen als Eingabeparameter eingerichtet.

Obwohl die vorliegende Erfindung anhand von Ausführungsbeispielen beschrieben wurde, ist sie vielfältig modifizierbar.

### BEZUGSZEICHENLISTE

- 2: Bauteil
- 3: Bauteil
- 4: Schweißnaht
- 4a: Schweißnaht
- 4b: Schweißnaht
- 5a: Kerbe
- 5b: Kerbe
- 5c: Kerbe
- 6a: finites Element
- 6b: finites Element
- 6c: finites Element
- 30: Finite-Elemente-Modell
- 7a: Koppelelement
- 7b: Koppelelement
- 7c: Koppelelement
- 8a: Netzmuster
- 8b: Netzmuster
- 8c: Netzmuster
- 9a: T-Stöße
- 9b: Stumpf-Stöße
- 9c: Überlapp-Stöße
- 10: Baugruppe
- 40: Finite-Elemente-Modell
- 50: Finite-Elemente-Modell
- 60: Finite-Elemente-Modell
- 100: Vorrichtung
- 101: erste Einheit
- 102: zweite Einheit
- 103: dritte Einheit
- S1: Verfahrensschritt
- S2: Verfahrensschritt
- S3: Verfahrensschritt

### REFERENZEN:

[1] IIW-Richtlinie: "Recommendations for Fatigue Design of Welded Joints and Components" vom International Institute of Welding (IIW) bzw. A. F. Hobbacher
[2] FKM-Richtlinie: "Rechnerischer Festigkeitsnachweis für Maschinenbauteile" vom Forschungskuratorium Maschinenbau (FKM) (Herausgeber)
[3] CN103838975A
[4] DE102012023670A1
[5] DE102014224129A1
[6] EP1337942B1
[7] EP3267338A1
[8] JP2003080393A
[9] US2013325417A1

## Patentansprüche

1. Computerimplementiertes Verfahren zur Modellerstellung und Festigkeitsbewertung von Schweißnähten (4a, 4b) zwischen mechanischen Bauteilen (2, 3) einer Baugruppe unter Zuhilfenahme einer Finite-Elemente-Methode, **gekennzeichnet durch**:
a) Bereitstellen (S1) eines Finite-Elemente-Modells (40, 50, 60) für die Baugruppe, bei welchem ein erstes Finite-Elemente-Netz für ein erstes Bauteil (2), ein getrenntes zweites Finite-Elemente-Netz für ein zweites Bauteil (3) und ein drittes Finite-Elemente-Netz für eine das erste (2) und das zweite Bauteil (3) verbindende Schweißnaht (4a, 4b) aufweisend eine Anzahl von Kerben (5a, 5b, 5c) erzeugt werden, wobei das dritte Finite-Elemente-Netz eine Anzahl von weniger als 20 finiten Elementen (6a, 6b, 6c) im Querschnitt aufweist, die Kerben (5a, 5b, 5c) der Schweißnaht (4a, 4b) dabei scharfkantig modelliert sind und die Aufteilung der finiten Elemente einem definierten Netzmuster (8a, 8b, 8c) folgt,
b) Berechnen (S2) des Finite-Elemente-Modells (40, 50, 60), wobei von dem definierten Netzmuster (8a, 8b, 8c) des dritten Finite-Elemente-Netzes für die Schweißnaht (4a, 4b) Ergebnisgrößen der finiten Elemente und Knoten bereitgestellt werden, und
c) Anwenden (S3) eines auf das definierte Netzmuster (8a, 8b, 8c) des dritten Finite-Elemente-Netzes abgestimmten Kerbspannungs-Prognosealgorithmus zur Prognose auftretender Kerbspannungen in den Kerben (5a, 5b, 5c) unter Verwendung der bereitgestellten Ergebnisgrößen als Eingabeparameter.

2. Verfahren nach Anspruch 1,
**dadurch gekennzeichnet,**
**dass** das erste Finite-Elemente-Netz und das dritte Finite-Elemente-Netz mittels einer ersten Anzahl von Koppelelementen (7a, 7b, 7c) gekoppelt werden und das zweite Finite-Elemente-Netz und das dritte Finite-Elemente-Netz mittels einer zweiten Anzahl von Koppelelementen (7a, 7b, 7c) gekoppelt werden.

3. Verfahren nach Anspruch 1 oder 2,
**dadurch gekennzeichnet,**
**dass** der Schritt b) (S2) ausgebildet ist durch:
b1) Berechnen des Finite-Elemente-Modells (40, 50, 60), und
b2) Auswerten der Ergebnisgrößen der finiten Elemente und der Knoten des definierten Netzmusters (8a, 8b, 8c) für die Schweißnaht (4a, 4b) auf der Basis des berechneten Finite-Elemente-Modells (40, 50, 60).

4. Verfahren nach Anspruch 3,
**dadurch gekennzeichnet,**
**dass** in dem Schritt b2) Ergebnisgrößen ausschließlich innerhalb der finiten Elemente und den Knoten des dritten Finite-Elemente-Netzes für die Schweißnaht (4a, 4b) ausgewertet werden.

5. Verfahren nach Anspruch 3 oder 4,
**dadurch gekennzeichnet,**
**dass** die Ergebnisgrößen, welche in dem Schritt b2) ausgewertet werden, umfassen:
- Spannungsergebnisse,
- Reaktionskraftergebnisse,
- Geometrieparameter, und/oder
- Materialparameter.

6. Verfahren nach Anspruch 3 oder 4,
**dadurch gekennzeichnet,**
**dass** die Ergebnisgrößen, welche in dem Schritt b2) ausgewertet werden, bestehen aus:
- Spannungsergebnisse,
- Reaktionskraftergebnisse,
- Geometrieparameter, und
- Materialparameter.

7. Verfahren nach einem der Ansprüche 1 bis 6,
**dadurch gekennzeichnet,**
**dass** der Kerbspannungs-Prognosealgorithmus vor der Anwendung des Schrittes c) mit einer Mehrzahl von Schweißnaht-Parametervarianten unter Verwendung des definierten Netzmusters (8a, 8b, 8c) trainiert wird.

8. Verfahren nach einem der Ansprüche 1 bis 7,
**dadurch gekennzeichnet,**
**dass** in dem Schritt c) (S3) eine Mehrzahl von Parametern der Kerben (5a, 5b, 5c) mittels des Kerbspannungs-Prognosealgorithmus prognostiziert wird.

9. Verfahren nach Anspruch 8,
**dadurch gekennzeichnet,**
**dass** die Parameter umfassen:
- Hauptnormalspannungen,
- Schubspannungen,
- Von-Mises-Vergleichsspannungen,
- Radial-Tangential-Spannungskomponenten, und/oder
- Axial-Spannungskomponenten im Kerbradius der jeweiligen Kerbe (5a, 5b, 5c).

10. Verfahren nach einem der Ansprüche 1 bis 9,
**dadurch gekennzeichnet,**
**dass** mit den prognostizierten Kerbspannungen anschließend Festigkeitsbewertungen der Baugruppe durchgeführt werden.

11. Computerprogrammprodukt, welches auf einer programmgesteuerten Einrichtung die Durchführung des Verfahrens nach einem der Ansprüche 1 bis 10 veranlasst.

12. Computerimplementierte Vorrichtung (100) zur Modellerstellung und Festigkeitsbewertung von Schweißnähten (4a, 4b) zwischen mechanischen Bauteilen (2, 3) einer Baugruppe unter Zuhilfenahme einer Finite-Elemente-Methode, **gekennzeichnet durch**:
eine erste Einheit (101) zum Bereitstellen eines Finite-Elemente-Modells (40, 50, 60) für die Baugruppe, bei welchem ein erstes Finite-Elemente-Netz für ein erstes Bauteil (2), ein getrenntes zweites Finite-Elemente-Netz für ein zweites Bauteil (3) und ein drittes Finite-Elemente-Netz für eine das erste (2) und das zweite Bauteil (3) verbindende Schweißnaht (4a, 4b) aufweisend eine Anzahl von Kerben (5a, 5b, 5c) erzeugt werden, wobei das dritte Finite-Elemente-Netz eine Anzahl von weniger als 20 finiten Elementen (6a, 6b, 6c) im Querschnitt aufweist, die Kerben (5a, 5b, 5c) der Schweißnaht (4a, 4b) dabei scharfkantig modelliert sind und die Aufteilung der finiten Elemente einem definierten Netzmuster (8a, 8b, 8c) folgt,
eine zweite Einheit (102) zum Berechnen des Finite-Elemente-Modells (40, 50, 60), wobei von dem definierten Netzmuster (8a, 8b, 8c) des dritten Finite-Elemente-Netzes für die Schweißnaht (4a, 4b) Ergebnisgrößen der finiten Elemente und Knoten bereitgestellt werden, und
eine dritte Einheit (103) zum Anwenden eines auf das definierte Netzmuster (8a, 8b, 8c) des dritten Finite-Elemente-Netzes abgestimmten Kerbspannungs-Prognosealgorithmus zur Prognose auftretender Kerbspannungen in den Kerben (5a, 5b, 5c) unter Verwendung der bereitgestellten Ergebnisgrößen als Eingabeparameter.

## Geänderte Patentansprüche

### Geänderte Patentansprüche gemäss Regel 137(2) EPÜ.

1. Computerimplementiertes Verfahren zur Modellerstellung und Festigkeitsbewertung von Schweißnähten (4a, 4b) zwischen mechanischen Bauteilen (2, 3) einer Baugruppe unter Zuhilfenahme einer Finite-Elemente-Methode, **gekennzeichnet durch**:
a) Bereitstellen (S1) eines Finite-Elemente-Modells (40, 50, 60) für die Baugruppe, bei welchem ein erstes Finite-Elemente-Netz für ein erstes Bauteil (2), ein getrenntes zweites Finite-Elemente-Netz für ein zweites Bauteil (3) und ein drittes Finite-Elemente-Netz für eine das erste (2) und das zweite Bauteil (3) verbindende Schweißnaht (4a, 4b) aufweisend eine Anzahl von Kerben (5a, 5b, 5c) erzeugt werden, wobei das dritte Finite-Elemente-Netz eine Anzahl von weniger als 20 finiten Elementen (6a, 6b, 6c) im Querschnitt aufweist, die Kerben (5a, 5b, 5c) der Schweißnaht (4a, 4b) dabei scharfkantig modelliert sind und die Aufteilung der finiten Elemente einem definierten Netzmuster (8a, 8b, 8c) folgt, wobei das erste Finite-Elemente-Netz und das dritte Finite-Elemente-Netz mittels einer ersten Anzahl von Koppelelementen (7a, 7b, 7c) gekoppelt werden und das zweite Finite-Elemente-Netz und das dritte Finite-Elemente-Netz mittels einer zweiten Anzahl von Koppelelementen (7a, 7b, 7c) gekoppelt werden,
b) Berechnen (S2) des Finite-Elemente-Modells (40, 50, 60), wobei von dem definierten Netzmuster (8a, 8b, 8c) des dritten Finite-Elemente-Netzes für die Schweißnaht (4a, 4b) Ergebnisgrößen der finiten Elemente und Knoten bereitgestellt werden,
c) Anwenden (S3) eines auf das definierte Netzmuster (8a, 8b, 8c) des dritten Finite-Elemente-Netzes abgestimmten Kerbspannungs-Prognosealgorithmus zur Prognose auftretender Kerbspannungen in den Kerben (5a, 5b, 5c) unter Verwendung der bereitgestellten Ergebnisgrößen als Eingabeparameter, und
d) Durchführen von Festigkeitsbewertungen der Baugruppe mit den prognostizierten Kerbspannungen.

2. Verfahren nach Anspruch 1,
**dadurch gekennzeichnet,**
**dass** der Schritt b) (S2) ausgebildet ist durch:
b1) Berechnen des Finite-Elemente-Modells (40, 50, 60), und
b2) Auswerten der Ergebnisgrößen der finiten Elemente und der Knoten des definierten Netzmusters (8a, 8b, 8c) für die Schweißnaht (4a, 4b) auf der Basis des berechneten Finite-Elemente-Modells (40, 50, 60).

3. Verfahren nach Anspruch 2,
**dadurch gekennzeichnet,**
**dass** in dem Schritt b2) Ergebnisgrößen ausschließlich innerhalb der finiten Elemente und den Knoten des dritten Finite-Elemente-Netzes für die Schweißnaht (4a, 4b) ausgewertet werden.

4. Verfahren nach Anspruch 2 oder 3,
**dadurch gekennzeichnet,**
**dass** die Ergebnisgrößen, welche in dem Schritt b2) ausgewertet werden, umfassen:
- Spannungsergebnisse,
- Reaktionskraftergebnisse,
- Geometrieparameter, und/oder
- Materialparameter.

5. Verfahren nach Anspruch 2 oder 3,
**dadurch gekennzeichnet,**
**dass** die Ergebnisgrößen, welche in dem Schritt b2) ausgewertet werden, bestehen aus:
- Spannungsergebnisse,
- Reaktionskraftergebnisse,
- Geometrieparameter, und
- Materialparameter.

6. Verfahren nach einem der Ansprüche 1 bis 5,
**dadurch gekennzeichnet,**
**dass** der Kerbspannungs-Prognosealgorithmus vor der Anwendung des Schrittes c) mit einer Mehrzahl von Schweißnaht-Parametervarianten unter Verwendung des definierten Netzmusters (8a, 8b, 8c) trainiert wird.

7. Verfahren nach einem der Ansprüche 1 bis 6,
**dadurch gekennzeichnet,**
**dass** in dem Schritt c) (S3) eine Mehrzahl von Parametern der Kerben (5a, 5b, 5c) mittels des Kerbspannungs-Prognosealgorithmus prognostiziert wird.

8. Verfahren nach Anspruch 7,
**dadurch gekennzeichnet,**
**dass** die Parameter umfassen:
- Hauptnormalspannungen,
- Schubspannungen,
- Von-Mises-Vergleichsspannungen,
- Radial-Tangential-Spannungskomponenten, und/oder
- Axial-Spannungskomponenten im Kerbradius der jeweiligen Kerbe (5a, 5b, 5c).

9. Computerprogrammprodukt, welches auf einer programmgesteuerten Einrichtung die Durchführung des Verfahrens nach einem der Ansprüche 1 bis 8 veranlasst.

10. Computerimplementierte Vorrichtung (100) zur Modellerstellung und Festigkeitsbewertung von Schweißnähten (4a, 4b) zwischen mechanischen Bauteilen (2, 3) einer Baugruppe unter Zuhilfenahme einer Finite-Elemente-Methode, **gekennzeichnet durch**:
eine erste Einheit (101) zum Bereitstellen eines Finite-Elemente-Modells (40, 50, 60) für die Baugruppe, bei welchem ein erstes Finite-Elemente-Netz für ein erstes Bauteil (2), ein getrenntes zweites Finite-Elemente-Netz für ein zweites Bauteil (3) und ein drittes Finite-Elemente-Netz für eine das erste (2) und das zweite Bauteil (3) verbindende Schweißnaht (4a, 4b) aufweisend eine Anzahl von Kerben (5a, 5b, 5c) erzeugt werden, wobei das dritte Finite-Elemente-Netz eine Anzahl von weniger als 20 finiten Elementen (6a, 6b, 6c) im Querschnitt aufweist, die Kerben (5a, 5b, 5c) der Schweißnaht (4a, 4b) dabei scharfkantig modelliert sind und die Aufteilung der finiten Elemente einem definierten Netzmuster (8a, 8b, 8c) folgt, wobei das erste Finite-Elemente-Netz und das dritte Finite-Elemente-Netz mittels einer ersten Anzahl von Koppelelementen (7a, 7b, 7c) gekoppelt werden und das zweite Finite-Elemente-Netz und das dritte Finite-Elemente-Netz mittels einer zweiten Anzahl von Koppelelementen (7a, 7b, 7c) gekoppelt werden,
eine zweite Einheit (102) zum Berechnen des Finite-Elemente-Modells (40, 50, 60), wobei von dem definierten Netzmuster (8a, 8b, 8c) des dritten Finite-Elemente-Netzes für die Schweißnaht (4a, 4b) Ergebnisgrößen der finiten Elemente und Knoten bereitgestellt werden,
eine dritte Einheit (103) zum Anwenden eines auf das definierte Netzmuster (8a, 8b, 8c) des dritten Finite-Elemente-Netzes abgestimmten Kerbspannungs-Prognosealgorithmus zur Prognose auftretender Kerbspannungen in den Kerben (5a, 5b, 5c) unter Verwendung der bereitgestellten Ergebnisgrößen als Eingabeparameter, und
eine vierte Einheit zum Durchführen von Festigkeitsbewertungen der Baugruppe mit den prognostizierten Kerbspannungen.
